# EUROPEAN PATENT APPLICATION

(11) **EP 1 229 323 A1**
(43) Date of publication of application: **07.08.2002**
(21) Application number: 02002220.8
(22) Date of filing: 30.01.2002
(51) Int. Cl.: G01N 21/89, G01N 33/36

(54) **Method of and device for detecting extraneous fibres in a yarn**

(30) Priority: 05.02.2001 CZ 20010440
(71) Applicant: Rieter CZ a.s., 562 15 Usti nad Orlici (CZ)
(72) Inventor: Stusák, Miroslav, 561 12 Brandys nad Orlici (CZ); Sloupensky, Jiri, 562 00 Usti nad Orlici (CZ)
(74) Representative: Bergmeier, Werner, Dipl.-Ing.

(57) **Abstract**

The invention relates to a method of detecting extraneous fibres and/or impurities in a yarn or in another linear fibre textile formation on an operating unit of a textile machine producing or processing yarn or another linear fibre textile formation. In this method, the colour picture of at least a part of the surface of the moving yarn (1) or of another linear fibre textile formation is at predetermined time intervals sensed (registered) and subject to a colour analysis aimed at detecting a spot of different colour in the colour picture of the yarn (1) or of another linear fibre textile formation representing a chromatic aberration due to the presence of an extraneous fibre or of impurities.

The device for carrying out the method comprises a radiation source (2) and a sensor (4) of the radiation reflected from the yarn (1) or another linear fibre textile formation whose output is connected with an evaluating device (5). The radiation sensor (4) consists of at least one row of colour-sensitive sensing elements (41).

## Description

### Technical field

The invention relates to a method of detecting extraneous fibres and/or impurities in a yarn or in another linear fibre textile formation on an operating unit of a textile machine producing or processing yarn or another linear fibre textile formation.

The invention also relates to a device for carrying out the method comprising a radiation source irradiating the yarn and a sensor of the radiation reflected from the yarn or from another linear fibre textile formation whose output is connected to an evaluating device.

### Background art

WO 97/36032 discloses a method of identification of extraneous matters in a yarn by means of a signal derived from the yarn and sorted in a sorting field. The sorting field contains as many threshold values as possible for the amplitude and length of the electric signal section. The threshold values assort the sorting field into classes each of which is defined by a combination of four threshold values. In the evaluation process, the electric signal derived from the yarn is continuously compared with all the threshold values as a basis for determining the class to which each yarn section belongs so as to assort yarn sections belonging to a given class. A signal deviation outside the set classes indicates the presence of an extraneous fibre, for instance of a jute fibre, and the affected yarn section must be cut out and removed.

Since the deviations of the signal derived from the yarn can be due also to other factors than extraneous matters in the yarn such as extraneous radiation sources, changes in the yarn surface quality etc., the reliability of the method and device described in WO 97/36032, in spite of the rather complicated evaluation it uses, is not great.

EP 553 446 A2 describes a method of detecting impurities, in particular extraneous fibres, in a yarn, in which there are registered a first signal whose value depends on the degree of the contamination by impurities of the textile formation, and a second signal, depending substantially only on the diameter and connected with the first signal in such a manner as to eliminate the dependence of the first signal on the diameter of the textile formation.

The drawback of the method consists in the necessity to use two sensors situated at a certain distance apart so that the comparison of the signals must take into consideration said distance and the yarn speed which increases the complicatedness of the evaluation, in particular at the start of the operating unit of the machine, for instance after the spinning-in. The reliability of the method of detecting extraneous fibres in the yarn disclosed in EP 553 446 A2 is low since the deviations of the first signal can be due not only to extraneous fibres or impurities in the yarn but also to other factors such as extraneous radiation sources, changes in the yarn surface quality etc.

EP 1 018 645 A1 discloses a device for detecting, by means of measuring and evaluating the reflected light, extraneous matters in a rope-like material such as fibre band or yarn with means for generating light and for irradiating the material. The means of generating the light and irradiating the material by the light has a light diode producing monochromatic colour light and a frequency transformer transforming the spectrum of the emitted light into white light. The means of measuring the reflected light, consisting of more than two sensors, is adapted to distinguish the colours on the basis of more than two colour reference points. Thus, if the means of measuring the reflected light comprise three sensors, the spectral sensitivity of each of them is set to a mutually different range of light colour. When registering the reflected light the sensors emit a signal proportional to the intensity of the colour range they have been set to. The evaluation consists in the measurement of the signal level of each sensor and in comparing the signal level with a set limit value. When the set limit value of at least one sensor is exceeded a signal is emitted indicating the presence of extraneous matters in the yarn.

The drawback of the device consists in particular in the complicatedness of the sensing device, the number of sensors, and the necessity to adjust each of them individually. This reduces the reliability both of the function of the device and of the evaluation, especially in the long run operation.
The invention intends to remove or at least to reduce the drawbacks of the background art and to create a method of reliable detection of extraneous fibres and/or impurities in the yarn or in another linear fibre formation. The invention also intends to create a simple and reliable device for carrying out the method.

### Principle of the invention

The goal of the invention has been reached by the method of detecting extraneous fibres and/or impurities in a yarn or in another linear fibre textile formation on an operating unit of a textile machine whose principle consists in that at predetermined time intervals the colour picture of at least a part of the surface of the moving yarn or of another linear fibre textile formation is sensed (registered) and subject to a colour analysis aimed at detecting a spot of different colour in the colour picture of the yarn or of another linear fibre textile formation representing a chromatic aberration due to the presence of an extraneous fibre or of impurities.

This method permits reliably to detect extraneous fibres in a yarn or in another linear fibre textile formation on an operating unit of a textile machine and subsequently to simplify the device for carrying out the method without the complicated setting and adjusting of the sensors.

The principle of the device for carrying out the method consists in that the radiation sensor consists of at least one row of colour-sensitive sensing elements arranged next to each other.

The output signal of such a sensor is the information on the colour picture of the yarn permitting to carry out the method according to the invention, i.e., to find out the spot of different colour in the colour picture of the yarn or of the linear fibre textile formation.

In the embodiment which permits the greatest yarn deflection in front of the radiation sensor, the position of the radiation sensor ensures that its sensing plane is perpendicular to the path of the yarn or of another linear fibre textile formation. Such arrangement is suitable in particular for sensing rather thick yarns.

In the device intended in particular for sensing rather thin yarns, the sensing plane of the radiation sensor makes an acute angle with the path of the yarn or of another linear fibre textile formation. Such arrangement imposes higher requirements on the exact course of the path of the yarn or of another linear fibre textile formation in front of the radiation sensor.

In each of the above described embodiments, the radiation source is preferably situated in front of the radiation sensor or after it so that the radiation source is situated sufficiently close to the sensed yarn or another linear fibre textile formation and thus reduces the risk of faults due to insufficient irradiation of the yarn or of another linear fibre textile formation.

The highest requirements on the precision of the yarn guiding are imposed by the device in which the front plane of the radiation sensor is in its longitudinal direction parallel with the path of the yarn or another linear fibre textile formation which means that the sensing plane passes through the yarn, in ideal case through the yarn axis. This embodiment covers the longest section of the moving yarn per one measurement interval with the ensuing increase in the reliability of the monitoring of yarn defects.

If the radiation properties are to be modified before its impact on the yarn, an optical member is inserted between the radiation source and the yarn or another linear fibre textile formation.

The modification of the properties of the radiation reflected from the yarn or another linear fibre textile formation can be achieved by inserting an auxiliary optical member in front of the radiation sensor.

As a rule, the radiation source is made as a general radiation source such as a LED diode. If a more exactly defined radiation and more exactly defined optical parameters are needed the radiation source is made as a single-point radiation source or as a parallel radiation source.

From the point of view of the simplicity of the device and of the cost reduction it is advantageous if the radiation source is made as a white light source.

Depending on the requirements imposed on the device, on the operating conditions, and on the special wishes presented by the customer on the price, quality, possibility of adjusting operation properties, etc., the radiation sensor is made as a colour-sensitive CMOS optical sensor or as a colour-sensitive CCD optical sensor.

In a preferred embodiment, the device contains at least two colour-sensitive radiation sources which permits to monitor a larger part of the surface of the yarn or of another linear fibre textile formation and thus to increase the precision of detecting extraneous fibres and/or impurities in the yarn or another linear fibre textile formation.

The possibility of monitoring the whole surface of the yarn or another linear fibre textile formation is afforded by the device comprising four sensors arranged around the surface of the yarn or another linear fibre textile formation and further comprising at least two radiation sources situated on mutually opposite sides of the yarn or another linear fibre textile formation. The monitoring of the whole surface of the yarn increases the qualitative properties of the device but also its cost of acquisition so that it is intended only for cases of high requirements placed upon the evaluation reliability.

A further increase in the reliability can be obtained by relating at least one radiation source to each radiation sensor so as to ensure a perfect irradiation of the yarn or another linear fibre textile formation.

### Description of the drawings

Examples of embodiment of the device for carrying out the method according to the invention are shown in the enclosed drawing in which Fig. 1 shows a device with a single-row radiation sensor arranged in a plane normal to the yarn path and with a single-point radiation source situated under the radiation sensor, Figs. 2 to 4 a device with a single-row radiation sensor arranged in a plane normal to the yarn path and with a single-point radiation source situated over the radiation sensor, Fig. 5 a device with a single-row radiation sensor arranged parallel to the yarn path and to a source of a parallel radiation arranged parallel with the yarn path, Fig. 6 a device with a single-row radiation sensor arranged in a plane making an acute angle with the yarn path, Fig. 7 a device with four radiation sensors situated around the yarn path and with two radiation sources, and Fig. 8 the course of the intensity of the irradiation of the sensor by the light reflected from the yarn.

### Examples of embodiment of the invention

The device for detecting extraneous fibres and/or impurities in the yarn or another linear fibre textile formation on an operating unit of a textile machine processing the yarn or another linear fibre textile formation shall be described on the example of a yarn producing rotor spinning machine.

The rotor spinning machine comprises a plurality of operating units situated next to each other each of which is fitted with means for yarn production and with means for winding the yarn on the bobbin. Each operating unit is equipped also with means for controlling and monitoring the function of the operating unit which means make a part of the control and information system of the operating unit and/or of a machine section and/or of the whole machine.

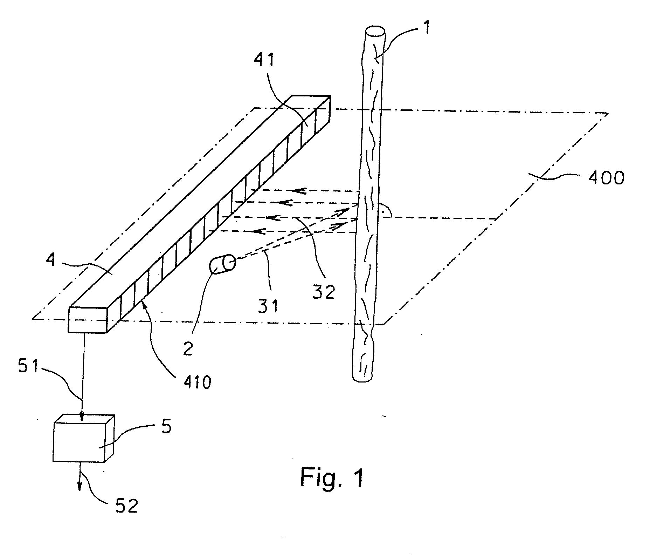

Coupled with the means for controlling and monitoring the function of the operating unit is a device for detecting extraneous fibres and/or impurities in the yarn situated at each operating unit of the machine close to the path of the yarn **1** being spun out, vertical in the illustrated examples of embodiment but not necessarily so since a general, not specified path of the yarn also can be used within the device according to the invention. The device shown in Fig. 1 comprises a radiation source **2** consisting in the shown example of embodiment of a single-point source of white light and situated next to the path of the yarn **1** being spun out. The radiation **31** emanating from it is directed to the yarn **1.** Situated over the radiation source **2** trsnsversely to the path of the yarn **1** is a colour-sensitive radiation sensor **4** consisting of at least one row of colour-sensitive sensing elements **41** oriented towards the yarn **1** and adapted to sense the radiation **32** reflected from the yarn **1.** The colour-sensitive sensing elements **41** make a front surface **410** of the radiation sensor **4.** The plane passing through the front surface **410** normal to it and parallel with its longer sides shall be referred to as sensing plane **400.** In this embodiment, the sensing plane **400** is normal to the path of the yarn **1.** In the area in front of the radiation sensor **4,** the yarn **1** is led by well-known not represented means such as guide rings ensuring its position in front of (opposite) the sensor **4** while leaving the yarn **1** free to move in a defined area in front of the radiation sensor **4** without negatively affecting the quality of sensing.

The output of the sensor **4** is led to the input **51** of an evaluating device **5** used to evaluate the information on the colour picture of the yarn **1** coming from the colour-sensitive sensing elements **41** of the radiation sensor **4** out of which the colour picture of the section of the surface of the monitored yarn **1** at a given moment or interval is composed.

The evaluating device **5** is fitted with an output **52** of information on the colour picture of the yarn which is connected with a not shown information and control system of the operating unit and/or of the machine section and/or of the machine.

The radiation source **2** sends toward the yarn **1** radiation **31** which is then reflected from the yarn surface. The radiation **32** reflected from the surface of the yarn **1** falls on the colour-sensitive sensing elements **41** of the sensor **4** which sensing elements **41** sense at predetermined each other following time intervals the colour picture of the section adjacent to the sensor **4** of the moving yarn **1.** In the evaluating device **5,** the colour picture of the yarn **1** undergoes a colour analysis in search of a yarn spot (section) of different colour indicating a colour defect of the yarn **1** due for instance to the presence of an extraneous fibre or of impurities.

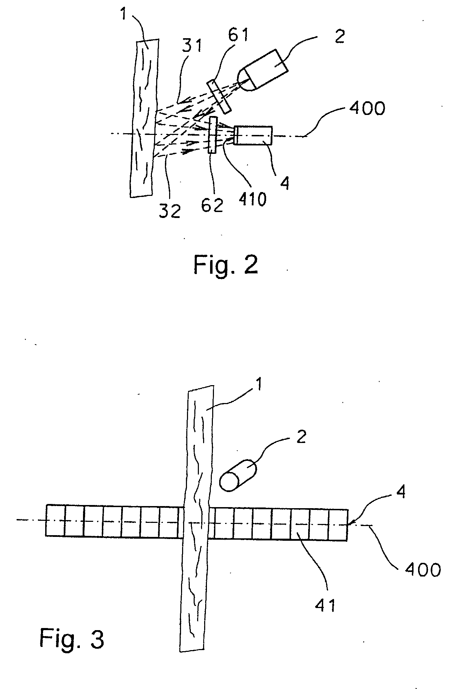

In the embodiment shown in Figs. 2 to 4, the radiation sensor **4** is situated under the radiation source **2**; as for the rest, the arrangement is analogical to that of Fig. 1. The embodiment shown in Fig.2 additionally contains a well-known optical member **61** put into the path of the radiation **31** coming from the radiation source **2** and serving to modify the radiation **31** before its impact on the surface of the yarn **1**, and a well-known auxiliary optical member **62** serving to modify the reflected radiation **32** before its impact on the colour-sensitive sensing elements **41** of the sensor **4**.

This embodiment is suitable in particular for thick yarns.

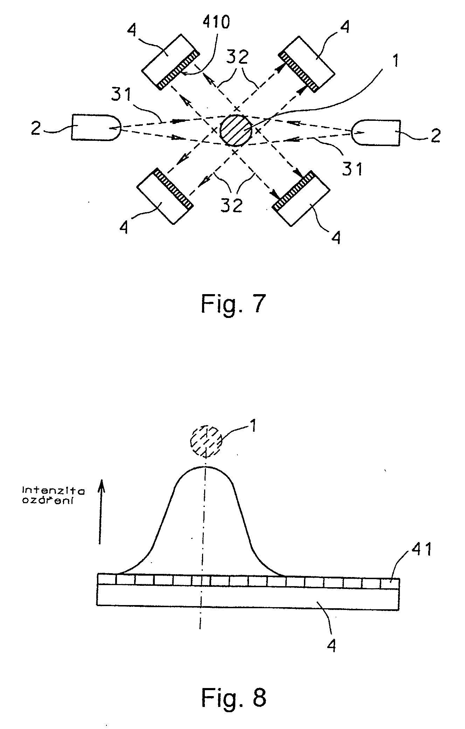

In sensing thin yarns, higher evaluating precision of the colour defects of the yarn can be obtained by using a number of single-row radiation sensors **4** in the arrangement shown in Figs. 1 to 4 over each other, or also by using a matrix radiation sensor, or by tilting the single-row radiation sensor **4** in relation to the yarn **1,** as is shown in Fig. 6 where the sensing plane **400** of the radiation sensor **4** makes an acute angle **&** with the path of the yarn **1.** As compared with the arrangement shown in Figs. 1 to 4 where the sensing plane **400** is normal to the path of the yarn **1,** the section of the monitored part of the surface of the yarn **1** during a single sensing interval is in this not illustrated embodiment longer and consequently the reliability of detecting extraneous fibres in the sensed yarn **1** greater. This arrangement is not limited to thin yarns but is applicable to strong yarns or other linear textile formations as well.

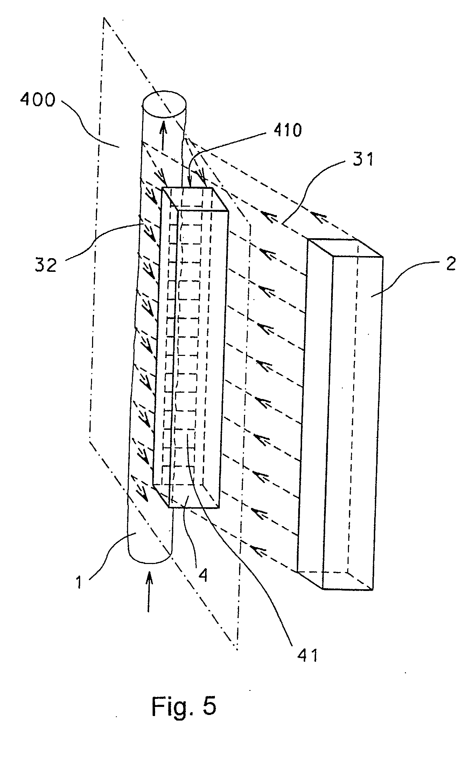

Another preferred embodiment of the device according to the invention, advantageous in particular for monitoring thin yarns, is shown in Fig. 5. In this embodiment, the front side **410** of the single-row radiation sensor **4** is in the longitudinal direction parallel with the direction of the motion of the yarn **1** or with the direction of the path of the yarn **1,** and has related thereto a source **2** of parallel radiation situated next to the sensor **4** parallel with the path of the yarn **1.** In this solution, the yarn **1** is sensed by the whole length of the radiation sensor **4** which means that all the colour-sensitive sensing elements transmit information on the colour picture of the yarn **1** situated in front of the radiation sensor **4** since during the sensing the yarn **1** must be situated in front of the radiation sensor **4** at its sensing angle. The sensing angle can be modified by means of the well-known auxiliary optical member **62** as shown in Fig. 2.

The light emitted by the radiation source **2** falls on the surface of the sensed yarn **1,** is reflected from it, and the reflected light is sensed by the radiation sensor **4.** During one sensing cycle, the sensor **4** reads the information on the intensity and colour of the impacting light and transmits it to the evaluating device **5** evaluating the colour picture of the sensed yarn **1** in search of deviations from the basic colour of the yarn **1** which is represented by the prevailing colour of the picture of the yarn **1** at a given measurement moment or is preset in a predetermined colour range. The basic colour can be set also as a colour combination which is advantageous in sensing multi-colour threads. If the colour picture of the yarn **1** furnishes information on the presence of a colour different from the basic one, the evaluating device sends out a signal or information on the presence of extraneous fibres or impurities and thus initiates the interruption of the yarn **1** or another reaction of the operating unit on the detected fault such as its evidence, illustration, etc.

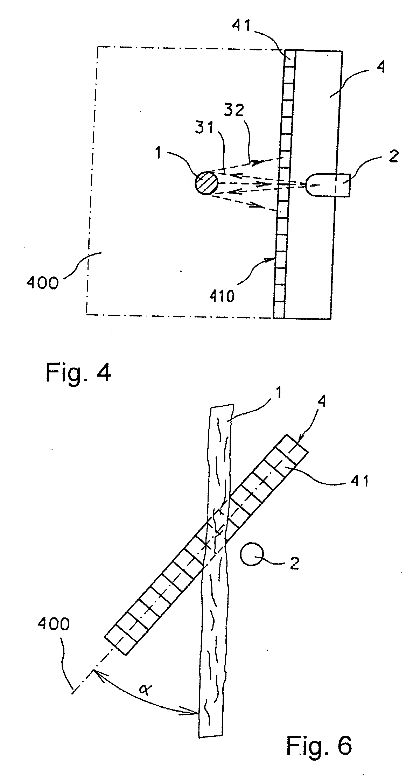

In the embodiment shown in Figs. 1 to 4 and 6, the radiation **32** reflected from the yarn **1** falls on the part of the colour-sensitive sensing elements **41** of the radiation sensor **4** situated opposite the yarn **1.** Due to the dispersion of the reflected radiation **32** the irradiated area is superior in size to the diameter of the yarn **1** as is shown in Fig. 4. The irradiation intensity of the sensing elements **41** of the radiation sensor **4** sinks with its increasing distance from the centre of the colour picture of the yarn **1** as shown in the diagram of Fig. 8.

The devices shown in Figs. 1 to 6 sense the yarn **1** at one of its sides so that they can sense only one half of the surface of the yarn **1** at the most. In most cases, such a sensing method is sufficient in view of the yarn motion and of the length of extraneous fibres which, as experience has proved, can be expected to appear on the whole circumference of the surface of the yarn **1.**

More problems are presented in searching for small impurities. To eliminate the drawback, the device has been elaborated into the embodiment shown in Fig. 7 fitted with two radiation sources **2** situated on opposite sides of the yarn **1,** i.e., substantially opposite each other, and emitting radiation **31** in the direction of (towards) the surface of the yarn **1** surrounded by four radiation sensors **4** receiving the radiation **32** reflected from the surface of the yarn **1.** The radiation sensors **4** consist of single-row (single-line) colour-sensitive optical sensors each of which furnishes information on the colour picture of its respective part of the surface of the yarn **1** and transmits it to the evaluating device **5** in which the information on the colour picture of the whole surface of the yarn **1** is produced. Within the information on the colour picture of the yarn **1** information on the presence of a colour different from the basic one is searched for.

It is also possible to monitor in the evaluating device **5** information on the colour picture of the yarn **1,** and to search for information on the presence of a colour different from the basic one, separately from each of the four radiation sensors **4.**

In the above described and illustrated examples of embodiment, the radiation sensor **4** consists of a single-row CMOS colour-sensitive optical sensor. In another embodiment, the radiation sensor **4** can consist of a single-row CCD colour-sensitive optical sensor or of a multi-row CMOS or CCD colour-sensitive optical sensor or of a matrix CMOS or CCD colour-sensitive optical sensor.

The invention is not limited to the described embodiments but extends also to the combinations and modifications thereof.

## Claims

1. A method of detecting extraneous fibres and/or impurities in a yarn or in another linear fibre textile formation on an operating unit of a textile machine producing or processing yarn or another linear fibre textile formation, **characterized by** that at predetermined time intervals the colour picture of at least a part of the surface of the moving yarn (1) or of another linear fibre textile formation is sensed (registered) and subject to a colour analysis aimed at detecting a spot of different colour in the colour picture of the yarn (1) or of another linear fibre textile formation representing a chromatic aberration due to the presence of an extraneous fibre or of impurities.

2. A device for carrying out the method claimed in Claim 1, comprising a radiation source (2) and a sensor (4) of the radiation reflected from the yarn or another linear fibre textile formation whose output is connected with an evaluating device (5), **characterized by** that the radiation sensor (4) consists of at least one row of colour-sensitive sensing elements (41).

3. A device as claimed in Claim 2, **characterized by** that the position of the radiation sensor (4) ensures that its sensing plane (400) is normal (perpendicular) to the path of the yarn (1) or of another linear fibre textile formation.

4. A device as claimed in Claim 2, **characterized by** that the position of the radiation sensor (4) ensures that its sensing plane (400) makes an acute angle (&) with the path of the yarn (1) or of another linear fibre textile formation.

5. A device as claimed in Claim 3 or 4, **characterized by** that the radiation source (2) is situated in front of the radiation sensor (4) or after it.

6. A device as claimed in Claim 2, **characterized by** that the front plane (410) of the radiation sensor (4) is in its longitudinal direction parallel with the path of the yarn (1) or another linear fibre textile formation.

7. A device as claimed in any of Claims 2 to 5, **characterized by** that an optical member (61) is inserted between the radiation source (2) and the yarn (1) or another linear fibre textile formation.

8. A device as claimed in any of Claims 2 to 7, **characterized by** that an auxiliary optical member (62) is inserted between the radiation sensor (4) and the yarn (1) or another linear fibre textile formation.

9. A device as claimed in any of Claims 2 to 8, **characterized by** that the radiation source (2) consists of a single-point radiation source.

10. A device as claimed in any of Claims 2 to 8, **characterized by** that the radiation source (2) consists of a parallel radiation source.

11. A device as claimed in any of Claims 2 to 10, **characterized by** that the radiation source (2) consists of a white light source.

12. A device as claimed in any of Claims 2 to 11, **characterized by** that the radiation sensor (4) consists of a colour-sensitive CCD sensor.

13. A device as claimed in any of Claims 2 to 11, **characterized by** that the radiation sensor (4) consists of a colour-sensitive CMOS sensor.

14. A device as claimed in any of Claims 2 to 13, **characterized by** that it contains at least two colour-sensitive radiation sensors (4).

15. A device as claimed in Claim 14, **characterized by** that it comprises four sensors (4) arranged around the surface of the yarn (1) or another linear fibre textile formation and at least two radiation sources (2) situated on mutually opposite sides of the yarn (1) or another linear fibre textile formation.

16. A device as claimed in Claim 14 or 15, **characterized by** that at least one radiation source (2) is related to each radiation sensor (2).
